# EUROPEAN PATENT APPLICATION

(11) **EP 1 752 133 A1**
(43) Date of publication of application: **14.02.2007**
(21) Application number: 05017435.8
(22) Date of filing: 11.08.2005
(51) Int. Cl.: A61K 8/81, A61K 8/49, A61Q 5/12

(54) **Use of polymeric thickeners in hair treating compositions, method and composition**

(71) Applicant: Wella Aktiengesellschaft, 64274 Darmstadt (DE)
(72) Inventor: Kolly-Hernandez, Maryline, 1762 Givisiez (CH); Kiener, Caroline, 1731 Ependes (CH); Mönks, Monika, 3185 Schmitten (CH)

(57) **Abstract**

The present invention provides the use of a thickening system to provide stable thickened hair treating composition, preferably hair care composition, that can provide enhanced touch, combability, alignment and volume reduction to the hair, with low or reduced greasy, oily hair feel, while being formulated having a thick viscosity with excellent spreading, perception of spreading, combability of hair and feel of hair.

## Description

### TECHNICAL FIELD

The invention relates to the use of polymeric thickeners to thicken hair treating compositions making the composition very stable against addition of ionic compounds, preferably cationic compounds like cationic salts.

### BACKGROUND OF THE INVENTION

In the area of hair conditioning compositions there is need for new thickening systems which are stable against the addition of ionic compounds such as e. g. salts, i. e. do not change viscosity.

Shampooing the hair removes excess sebum and other environmental soiling but has disadvantages in that the hair can be left in a wet, tangled and relatively unmanageable state. Shampooing can also result in the hair becoming dry due to the removal of natural oils or other hair moisturizing materials. After shampooing, the hair can also suffer from a perceived loss of softness. Frequent shampooing also contributes to the phenomena of "split ends," particularly for long hair. Split ends refers to a condition wherein the ends of the hair are split into two or more shafts, resulting in a frizzy appearance.

A variety of approaches have been developed to condition the hair. These range from post-shampooing hair rinses, to leave-on hair conditioners, to the inclusion of hair conditioning components in shampoos. Although many consumers prefer the ease and convenience of a shampoo which includes conditioners, a substantial proportion prefer the more conventional conditioner formulations which are applied to the hair as a separate step from shampooing, usually subsequent to shampooing. These hair conditioners typically are formulated as a thickened product, such as a gel or cream, for ease of dispensing and application to the hair.

Hair rinse conditioners have most conventionally been based on the combination of a cationic surfactant, which is generally a quaternary ammonium compound such as ditallow dimethyl ammonium chloride, and fatty alcohols, such as cetyl and stearyl alcohols. This combination results in a gel-network structure which provides the compositions with a thick, creamy rheology but which could be unstable during storage over longer periods of time and/or at higher temperatures.

It is therefore an object of this invention to provide the use of a new thickening system to thicken hair treating compositions without having the tendency to be unstable during storage. It is a further object of this invention to provide the use of a new thickening system to thicken hair treating compositions which does nor render to be unstable by addition of ionic compounds. It is desirable to provide such a composition, as described above in a thickened form, such as a gel, which can be easily applied and rinsed from the hair. It is a further object of this invention to provide such a hair care composition that has an aesthetically pleasing wet hair feel, a glossy consistency and perception of spreading upon application to the hair together with excellent wet and dry combability of hair. It is further desirable to provide a method for conditioning hair in accordance with the above compositions.

These and other objects and benefits of the present invention as may be set forth herein as may now or later become apparent to those skilled in the art can be provided according to the invention which is described herein.

The invention hereof can comprise, consist of, or consist essentially of the essential elements described herein as well as any of the preferred or other optional ingredients described herein.

All percentages herein are by weight of the composition unless otherwise indicated. All ratios are weight ratios unless otherwise indicated. Unless otherwise indicated, all percentages, ratios, and levels of ingredients referred to herein are based on the actual amount of the ingredient, and do not include solvents, fillers, or other materials with which the ingredient may be combined in commercially available products. All documents referred to herein, including all patents, all patent applications and all articles, are hereby incorporated herein by reference in their entirety.

### SUMMARY OF THE INVENTION

The present invention provides stable thickened hair treating compositions, preferably hair care compositions, that can provide enhanced touch, combability, alignment and volume reduction to the hair, with low or reduced greasy, oily hair feel, while being formulated in an emulsion having a more or less thick, creme- or gel-type rheology with excellent spreading, perception of spreading, and feel.

In particular, the present invention refers to the use of a thickening system that comprises a mixture of
(i) from about 50 to about 70% of a copolymer of 2-propenoic acid with 2-propenamide (CAS 26100-47-0);
(ii) from about 25 to about 35% of the homopolymer of 2-methyl-1-propene (polyisobutene) (CAS 9003-27-4; CTFA: POLYISOBUTENE);
(iii) from about 3 to about 8% of poly(oxy-1,2-ethanediyl)-sorbitan-monododecanoate (CAS 9005-64-5; CTFA: POLYSORBATE-20) and
(iv) from 0 to about 22% by weight of water
in an amount of from about 0.1 % to about 30% by weight, preferably about 0.5 to about 10% by weight, to thicken hair treating compositions, preferably hair conditioning compositions.

The present invention also provides a hair conditioning composition that comprises
(A) a thickening system that comprises a mixture of
   (i) from about 50 to about 70% of a copolymer of 2-propenoic acid with 2-propenamide (CAS 26100-47-0);
   (ii) from about 25 to about 35% of the homopolymer of 2-methyl-1-propene (polyisobutene) (CAS 9003-27-4; CTFA: POLYISOBUTENE);
   (iii) from about 3 to about 8% of poly(oxy-1,2-ethanediyl)-sorbitan-monododecanoate (CAS 9005-64-5; CTFA: POLYSORBATE-20) and
   (iv) from 0 to about 22% by weight of water,
   in an amount of from about 0.1 % to about 30% by weight of the total weight of the hair conditioning composition and
(B) a hair conditioning agent in an amount of from about 0.1 % to about 40% by weight, preferably from about 0.5 % to about 10% by weight, of the total weight of the hair conditioning composition.

The present invention also provides a method for conditioning hair by application to the hair of an effective amount of the compositions hereof to enhance glossiness of the hair.

### DETAILED DESCRIPTION OF THE INVENTION

The essential ingredients of the hair treating composition, which preferably is a hair conditioning composition, as well as a variety, but non-exclusive, list of preferred and optional ingredients are described below.

### Thickening system

The thickening system preferably comprises a mixture of
(a) from about 60 to about 67% of a copolymer of 2-propenoic acid with 2-propenamide (CAS 26100-47-0);
(b) from about 27 to about 32% of the homopolymer of 2-methyl-1-propene (CAS 9003-27-4; CTFA: POLYISOBUTENE);
(c) from about 5 to about 7% of poly(oxy-1,2-ethanediyl)-sorbitan-monododecanoate (CAS 9005-64-5; CTFA: POLYSORBATE-20) and
(d) from 0 to about 22% by weight of water.

A thickening system according to the invention is sold under the trade name Sepiplus^{®} 265, by SEPPIC Inc., USA.

The hair treating composition preferably contains from about 0.5 to about 20% by weight, more preferably from about 0.5 to about 10% by weight, and most preferably from about 1.0 to about 5% by weight of the thickening system.

### Water

The thickening system of the invention preferably contains about 1 to about 10% by weight of the thickening system of water, whereas the hair treating composition preferably contains about 50 to about 98% by weight, more preferably from about 60 to about 96% by weight, and most preferably from about 70 to about 95% by weight, of the total weight of the hair treating composition of water.

### Other solvents

The hair treating composition can optionally include other liquid, water-miscible or water-soluble solvents such as lower alkyl alcohols, e. g. C1-C5 alkyl monohydric alcohols, preferably C2-C3 alkyl alcohols, most preferred ethanol or isopropanol. The water-soluble polyhydric alcohols usable in the present invention are also polyhydric alcohols having two or more hydroxyl groups in the molecule. Typical examples of such polyhydric alcohols are dihydric alcohols such as ethylene glycol, propylene glycol; trimethylene glycol, 1,2-butylene glycol, 1,3-butylene glycol, 1,4-butylene glycol, tetramethylene glycol, 2,3-butylene glycol, pentamethylene glycol, 2-butene-1,4-diol, hexylene glycol, octylene glycol; trihydric alcohols such as glycerine, trimethylol propane, 1,2,6-hexanetriol and the like; tetrahydric alcohols such as penthaerythritol; pentahydric alcohols such as xylytol, etc.; hexahydric alcohols such as sorbitol, mannitol; polyhydric alcohol polymers such as diethylene glycol, dipropylene glycol, polyethylene glycol, polypropylene glycol, tetraethylene glycol, diglycerine, polyethylene glycol, triglycerine, tetraglycerine, polyglycerine; dihydric alcohol alkyl ethers such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether; dihydric alcohol alkyl ethers such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether; dihydric alcohol ether esters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate; glycerine monoalkyl ethers such as xyl alcohol, selachyl alcohol, batyl alcohol; sugar alcohols such as sorbitol, maltitol, maltotriose, mannitol, sucrose, erythritol, glucose, fructose, starch sugar, maltose, xylytose, starch sugar reduced alcohol, glysolid, tetrahydrofurfuryl alcohol, POE tetrahydrofurfuryl alcohol, POP butyl ether, POP POE butyl ether, tripolyoxypropylene glycerine ether, POP glycerine ether, POP glycerine ether phosphoric acid, POP POE pentanerythritol ether.

The hair treating composition optionally preferably contains about 0.5 to about 30% by weight, more preferably from about 1.0 to about 20% by weight, and most preferably from about 1.0 to about 15% by weight of the other solvent.

The hair conditioning agent is selected from the group of cationic surfactants, cationic polymers, silicone compounds and other conditioning agents or mixtures thereof as specified below:

### Cationic Surfactant Conditioning Agents

Cationic surfactants that can be preferably used in the composition of the present invention contain amino or quaternary ammonium moieties. Cationic surfactants among those useful herein are disclosed in the following documents, all incorporated by reference herein: U. S. Patent 3,929,678, Laughlin et al., issued December 30, 1975; U S. Patent 3,959,461, Bailey et al., issued May 25, 1976; and U. S. Patent 4,387,090, Bolich, Jr., issued June 7, 1983.

Among the quaternary ammonium-containing cationic surfactant materials useful herein are those of the general formula (I)

[NR1,R2,R3,R4]⁺· X⁻

wherein R1 to R4 are independently an aliphatic group of from about 1 to about 22 carbon atoms or an aromatic, alkoxy, polyoxyalkylene, alkylamido, hydroxyalkyl, aryl or alkylaryl group having from about 1 to about 22 carbon atoms; and X⁻ is a salt-forming anion such as those selected from halogen, (e. g. chloride, bromide, iodide), acetate, citrate, lactate, glycolate, phosphate nitrate, sulfate, and alkylsulfate radicals.

The aliphatic groups may contain, in addition to carbon and hydrogen atoms, either linkages, and other groups such as amino groups. The longer chain aliphatic groups, e. g. those of about 12 carbons, or higher, can be saturated or unsaturated. Especially preferred are di-long chain (e.g., di C12-C22, preferably C16-C18, aliphatic, preferably alkyl), di-short chain (e.g. C1 - C3 alkyl, preferably C1 - C2 alkyl) ammonium salts. Salts of primary, secondary and tertiary fatty amines are also suitable cationic surfactant materials. The alkyl groups of such amines preferably have from about 12 to about 22 carbon atoms, and may be substituted or unsubstituted. Such amines, useful herein, include stearamido propyl dimethyl amine, diethyl amino ethyl ste-aramide, dimethyl stearamine, dimethyl soyamine, soyamine, myristyl amine, tridecyl amine, ethyl stearylamine, N-tallowpropane diamine, ethoxylated (5 moles E.O.) stearylamine, dihydorxy ethyl stearylamine, and arachidylbehenylamine. Suitable amine salts include the halogen, acetate, phosphate, nitrate, citrate, lactate, and alkyl sulfate salts. Such salts include stearylamine hydrochloride, soyamine chloride, stearylamine formate, N-tallowpropane diamine dichloride and stearamidopropyl dimethylamine citrate. Preferred cationic surfactants are cetyl trimethyl ammonium salts, as for example Genamin^{®} CTAC, i. e. cetyl trimethyl ammonium chloride, behenyl trimethyl ammonium salts, e. g. behenyl trimethyl ammonium chloride; dimethyl ditallow ammonium salts; stearyl amidopropyl dimethylamine; esterquats as for example tetradecyl betainester chloride, diesterquats as for example dipalmitylethyl - dimethylammoniumchloride (Armocare^{®} VGH70 of Akzo, Germany), or a mixture of distearoylethyl hydroxyethylmonium methosulfate and Cetearyl Alkohol (Dehyquart^{®} F-75 of Henkel, Germany).

Cationic surfactants are preferably contained at levels of from about 0.1 % to about 5%, more preferably from about 0.2% to about 1,5%, most preferably from about 0.4% to about 0.8%, by weight of the composition.

### Cationic Polymer Conditioning Agents

The compositions of the present invention can also contain one or more cationic polymer as conditioning agents. The cationic polymer conditioning agent will preferably be water soluble. Cationic polymers are typically used in the same ranges as disclosed above for cationic surfactants.

By "water soluble" cationic organic polymer, what is meant is a polymer which is sufficiently soluble in water to form a substantially clear solution to the naked eye at a concentration of 0.1 % in water (distilled or equivalent) at 25°C. Preferably, the polymer will be sufficiently soluble to form a substantially clear solution at 0.5% concentration, more preferably at 1.0% concentration. As used herein, the term "polymer" shall include materials whether made by polymerization of one type of monomer or made by two (i.e., copolymers) or more types of monomers.

The cationic polymers hereof will generally have a weight average molecular weight which is at least about 5,000, typically at least about 10,000, and is less than about 10 million. Preferably, the molecular weight is from about 100,000 to about 2 million. The cationic polymers will generally have cationic nitrogen-containing moieties such as quaternary ammonium or cationic amino moieties, or a mixture thereof.

The cationic charge density is preferably at least about 0.1 meq/gram, more preferably at least about 1.5 meq/gram, even more preferably at least abut 1.1 meq/gram, most preferably at least about 1.2 meq/gram. Cationic charge density of the cationic polymer can be determined according to the Neldahl Method. Those skilled in the art will recognize that the charge density of amino-containing polymers may vary depending upon pH and the isoelectric point of the amino groups. The charge density should be within the above limits at the pH of intended use. Any anionic counterions can be utilized for the cationic polymers so long as the water solubility criteria is met. Suitable counterions include halides (e. g., Cl, Br, I, or F, preferably Cl, Br, or I), sulfate, and methylsulfate. Others can also be used, as this list is not exclusive. The cationic nitrogen-containing moiety will be present generally as a substituent, on a fraction of the total monomer units of the cationic hair conditioning polymers. Thus, the cationic polymer can comprise copolymers, terpolymers etc. of quaternary ammonium or cationic amine-substituted monomer units and other non-cationic units referred to herein as spacer monomer units.

Suitable cationic polymers include, for example, copolymers of vinyl monomers having cationic amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone, and vinyl pyrrolidone. The alkyl and dialkyl substituted monomers preferably have C1-C7 alkyl groups, more preferably C1-C3 alkyl groups.

Other suitable spacer monomers include vinyl esters, vinyl, alcohol (made by hydrolysis of polyvinyl acetate), maleic anhydride, propylene glycol, and ethylene glycol.

The cationic amines can be primary, secondary, or tertiary amines, depending upon the particular species and the pH of the composition. In general, secondary and tertiary amines, especially tertiary, amines, are preferred. Amine-substituted vinyl monomers can be polymerized in the amine form, and then optionally can be converted to ammonium by a quaternization reaction. Amines can also be similarly quaternized subsequent to formation of the polymer. For example, tertiary amine functionalities can be quaternized by reaction with a salt of the formula R'X wherein R' is a short chain alkyl, preferably a C1-C7 alkyl, more preferably a C1-C3 alkyl, and X is an anion which forms a water soluble salt with the quaternized ammonium.

Suitable cationic amino and quaternary ammonium monomers include, for example, vinyl compounds substituted with dialkylaminoalkyl acrylate, dialkylaminoalkyl methacrylate, monoalkylaminoalkyl acrylate, monoalkylaminoalkyl methacrylate, trialkyl methacryloxyalkyl ammonium salt, trialkyl acryloxyalkyl ammonium salt, diallyl quaternary ammonium salts, and vinyl quaternary ammonium monomers having cyclic cationic nitrogen-containing rings such as pyridinium, imidazolium, and quaternized pyrrolidone, e. g. alkyl vinyl imidazolium, alkyl vinyl pyridinium, alkyl vinyl pyrrolidone salts. The alkyl portions of these monomers are preferably lower alkyls such as the C1-C3 alkyls, more preferably C1 and C2 alkyls. Suitable amine-substituted vinyl monomers for use herein include dialkylaminoalkyl acrylate, dialkylaminoalkyl methacrylate, dialkylaminoalkyl acrylamide, and dialkylaminoalkyl methacrylamide, wherein the alkyl groups are preferably C1-C7 hydrocarbyls, more preferably C1-C3 alkyls.

The cationic polymers hereof can comprise mixtures of monomer units derived from amine- and/or quaternary ammonium-substituted monomer and/or compatible spacer monomers. Suitable cationic hair conditioning polymers include, for example: co-polymers of 1-vinyl-2-pyrrolidone and 1-vinyl-3-methylimidazolium salt (e. g. chloride salt) (referred to in the industry by the Cosmetic, Toiletry, and Fragrance Association, "CTFA", as POLYQUATERNIUM-16), such as those commercially available from BASF Wyandotte Corp. (Parsippany, NJ, USA) under the LUVIQUAT tradename (e. g. LUVIQUAT FC 370); copolymers of 1-vinyl-2-pyrrolidone and dimethylaminoethyl methacrylate (referred to in the industry by CTFA as POLYQUATERNIUM-11) such as those commercially available from Gaf Corporation (Wayne, NJ, USA) under the GAFQUAT tradename (e.g., GAFQUAT 755N); cationic diallyl quaternary ammonium-containing polymers, including, for example, dimethyl diallyl ammonium chloride homopolymer and copolymers of acrylamide and dimethyl diallyl ammonium chloride, referred to in the industry (CTFA) as POLYQUATERNIUM-6 and POLYQUATERNIUM-7, respectively; and mineral acid salts of amino-alkyl esters of homo- and co-polymers of unsaturated carboxylic acids having from 3 to 5 carbon atoms, as described in U.S. Patent 4,009,256, incorporated herein by reference. Other cationic polymers that can be used include polysaccharide polymers, such as cationic cellulose derivatives and cationic starch derivatives. Cationic polysaccharide polymer materials suitable for use herein include those of the formula (II): 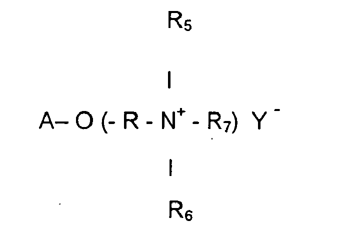 wherein: A is an anhydroglucose residual group, such as a starch or cellulose anhydroglucose residual, R is an alkyene, oxyalkylene, polyoxyalkylene or hydroxyalkylene group, or combination thereof, R₅, R₆, and R₇ independently are alkyl, aryl, alkylaryl, arylalkyl, alkoxyalkyl, or alkoxyaryl groups, each group containing 1 to about 18 carbon atoms, and the total number of carbon atoms for each cationic moiety (i. e. the sum of carbon atoms in R₅, R₆ and R₇) preferably being about 20 or less, and Y is an anionic counterion, as previously described.

Cationic cellulose is available from Amerchol Corp. (Edison, NJ, USA) in their Polymer JR^{®} and LR^{®} series of polymers, as salts of hydroxyethyl cellulose reacted with trimethyl ammonium substituted epoxide, referred to in the industry (CTFA) as POLYQUATERNIUM-10.

Another type of cationic cellulose includes the polymeric quaternary ammonium salts of hydroxyethyl cellulose reacted with lauryl dimethyl ammonium-substituted epoxide, referred to in the industry (CTFA) as POLYQUATERNIUM-24. These materials are available from Amerchol Corp. (Edison, NJ, USA) under the tradename Polymer LM-200.

Other cationic polymers that can be used include cationic quar gum derivatives, such as CTFA: GUAR HYDROXYPROPYL TRIMONIUM CHLORIDE (commercially available from Celanese Corp. in their JaguarR^{®} series).

Other materials include quaternary nitrogen-containing cellulose ethers (e. g. as described in U.S. Patent 3,962,418, incorporated by reference herein), and copolymers of etherified cellulose and starch (e. g. as described in U.S. Patent 3,958,581, incorporated by reference herein).

As discussed above, the cationic polymer hereof is water soluble. This does not mean, however, that it must be soluble in the composition. Preferably however, the cationic polymer is either soluble in the composition, or in a complex coacervate phase in the composition formed by the cationic polymer and anionic material. Complex coacervates of the cationic polymer can be formed with anionic surfactants or with anionic polymers that can optionally be added to the compositions hereof (e. g. sodium polystyrene sulfonate).

The preferred cationic polymers are CTFA: QUATERNIUM-10 and GUAR HYDROXYPROPYL TRIMONIUM CHLORIDE.

The cationic polymer hair conditioning agent can be used in the compositions hereof at levels of from about 0.1 % to about 10% by weight of the composition, preferably from about 0.3% to about 5%, more preferably from about 0.3% to about 3%, most preferably from about 0.3% to about 1.0 % by weight.

### Silicone Conditioning Agents

The compositions hereof can also include volatile or nonvolatile, soluble or insoluble silicones as conditioning agents. By soluble what is meant is that the silicone conditioning agent is miscible with the aqueous carrier of the composition so as to form part of the same phase. By insoluble what is meant is that the silicone from a separate, discontinuous phase from the aqueous carrier, such as in the form of an emulsion or a suspension of droplets of the silicone.

Soluble silicones include silicone copolyols, such as dimethicone copolyols, e.g. polyether siloxane-modified polymers, such as polypropylene oxide, polyethylene oxide modified polydimethylsiloxane, wherein the level of ethylene and/or propylene oxide sufficient to allow solubility in the composition.

Preferred, however, are insoluble silicones. The insoluble silicone hair conditioning agent for use herein will preferably have viscosity of from about 1,000 to about 2,000,000 mPa · s at 25°C, more preferably from about 10,000 to about 1,800,000, even more preferably from about 100,000 to about 1,500,000 mPa · s at 25°C. The viscosity can be measured by means of a glass capillary viscometer as set forth in Dow Corning Corporate Test Method CTM0004, July 20, 1970.

Suitable insoluble, nonvolatile silicone fluids include polyalkyl siloxanes, polyaryl siloxanes, polyalkylaryl siloxanes, polyether siloxane copolymers, dimethylpolysiloxane containing terminal hydroxyl groups, methylphenyl polysiloxane containing terminal hydroxyl groups and mixtures thereof. Specific examples thereof include hexamethyldisiloxane, octamethyltrisiloxane, decamethyltetrasiloxane and hexadeca-methylheptasiloxane.

Other insoluble, nonvolatile silicone fluids having hair conditioning properties can also be used. The term "nonvolatile" as used herein shall mean that the silicone has a boiling point of at least about 260°C, preferably at least about 275°C, more preferably at least about 300°C. Such materials exhibit very low or no significant vapor pressure at ambient conditions. The term "silicone fluid" shall mean flowable silicone materials having a viscosity of less than 1,000,000 mPa · s at 25°C. Generally, the viscosity of the fluid will be between about 5 and 1,000,000 mPa · s at 25°C, preferably between about 10 and about 300,000 mPa · s at 25°C.

The preferred silicones are polydimethyl siloxane, polydiethylsiloxane, and polymethylphenylsiloxane. Polydimethylsiloxane is especially preferred. The nonvolatile polyal-kylsiloxane fluids that may be used include, for example, polydimethylsiloxanes. These siloxanes are available, for example, from the General Electric Company in their ViscasilR and SF 96 series, and from Dow Corning in their Dow Corning 200^{®} series.

The polyalkylaryl siloxane fluids that may be used, also include, for example, polymethylphenylsiloxanes. These siloxanes are available, for example, from the General Electric Company as SF 1075 methyl phenyl fluid or from Dow Corning as 556 Cosmetic Grade Fluid or diquaternary silikones as for example CTFA: QUATERNIUM-80 (e. g. Abil^{®} Quat 3272 or Abil^{®} Quat 3270 of Th. Goldschmidt AG, Germany).

Suitable insoluble, volatile silicone fluids include low molecular weight oligomeric polydimethylsiloxane or cyclic polydimethylsiloxane, having a viscosity of no more than 10 mPa · s at 25°C and a boiling point under atmospheric pressure of no more than 250°C. Volatility can be achieved in linear organopolysiloxanes by selection of oligomeric organopolysiloxanes with at most 6 to 10 silicone atoms in the organopolysiloxanes backbone, e. g. Dow Corning DC200 Fluid, having a viscosity of from about 0.65 to about 2 mPa · s at 25°C. Preferably cylclic organopolysiloxanes having from 3 to 6 silicon atoms are utilized, for example, hexamethylcyclotrisiloxane, octamethylcyclotetrasiloxane, decamethylcyclopentasiloxane, dodecamethylcyclohexasiloxane (e. g. DC 244, DC 245, DC 345 of Dow Corning).

Especially preferred, for enhancing the shine characteristics of hair, are highly arylated silicones, such as highly phenylated polyethyl silicone having refractive indices of about 1.46 or higher, especially about 1.52 or higher. When these high refractive index silicones are used, they should be mixed with a spreading agent, such as a surfactant or a silicone resin, as described below to decrease the surface tension and enhance the film forming ability of the material.

The polyether siloxane copolymers that may be used include, for example, a polypropylene oxide modified polydimethylsiloxane (e. g. Dow Corning DC-1248^{®}) although ethylene oxide or mixtures of ethylene oxide and propylene oxide may also be used. The ethylene oxide and polypropylene oxide level should be sufficiently low to prevent solubility in the composition hereof.

Another silicone hair conditioning material that can be especially useful in the silicone conditioning agents is insoluble silicone gum. The term "silicone gum", as used herein, means polyorganosiloxane materials having a viscosity at 25°C of greater than or equal to 1,000,000 mPa · s. Silicone gums are described by Petrarch and others including U.S. Patent 4,152,416. These described references are incorporated herein by reference. The "silicone gums" will typically have a mass molecular weight in excess of about 200,000, generally between about 200,000 and about 1,000,000. Specific examples include polydimethylsiloxane, (polydimethylsiloxane) (methylvinylsiloxane) copolymer, poly(dimethylsiloxane) (diphenyl siloxane) (methylvinylsiloxane) copolymer and mixtures thereof. Preferably the silicone hair treating agent comprises a mixture of a polydimethylsiloxane gum, having a viscosity greater than about 1,000,000 mPa · s and polydimethylsiloxane fluid having a viscosity of from about 10 mPa · s to about 100,000 mPa · s at 25°C, wherein the ratio of gum to fluid is from about 30:70 to about 70:30, preferably from about 40:60 to about 60:40.

An optional ingredient that can be included in the silicone conditioning agent is silicone resin. Silicone resins are highly crosslinked polymeric siloxane systems. The crosslinking is introduced through the incorporation of trifunctional and tetrafunctional silanes with monofunctional or difunctional, or both, silanes during manufacture of the silicone resin. As is well understood in the art, the degree of crosslinking that is required in order to result in a silicone resin will vary according to the specific silane units incorporated into the silicone resin. In general, silicone materials which have a sufficient level of trifunctional and tetrafunctional siloxane monomer units (and hence, a sufficient level of crosslinking) such that they dry down to a rigid, or hard, film are considered to be silicone resins. The ratio of oxygen atoms to silicon atoms is indicative of the level of crosslinking in a particular silicone material. Silicone materials which have at least about 1.1 oxygen atoms per silicon atom will generally be silicone resins herein.

Preferably, the ratio of oxygen : silicon atoms is at least about 1.2 :1.0. Silanes used in the manufacture of silicone resins include monomethyl-, dimethyl-, trimethyl-, monophenyl-, diphenyl-, methylphenyl-, monovinyl-, and methylvinyl-chlorosi lanes, and tetrachlorosilane, with the methyl- substituted silanes being most commonly utilized. Preferred resins are offered by General Electric as GE SS4230 and SS4267^{®}. Commercially available silicone resins will generally be supplied in a dissolved form in a low viscosity volatile or nonvolatile silicone fluid. The silicone resins for use herein should be supplied and incorporated into the present compositions in such dissolved form, as will be readily apparent to those skilled in the art. Silicone resins can enhance deposition of silicone on the hair and can enhance the glossiness of hair with high refractive index volumes.

Silicone materials and silicone resins in particular, can conveniently be identified according to a shorthand nomenclature system well known to those skilled in the art as "MDTQ" nomenclature. Under this system, the silicone is described according to presence of various siloxane monomer units which make up the silicone. Briefly, the symbol M denotes the monofunctional unit (CH3)3SiO).5; D denotes the difunctional unit (CH3)2SiO; T denotes the trifunctional unit (CH3)SiO1.5; and Q denotes the quadri- or tetra-functional unit SiO2. Primes of the unit symbols, e.g., M', D', '17, and Q' denote substituents other than methyl, and must be specifically defined for each occurrence. Typical alternate substituents include groups such as vinyl, phenyls, amines, hydroxyls, etc. The molar ratios of the various units, either in terms of subscripts to the symbols indicating the total number of each type of unit in the silicone (or an average thereof) or as specifically indicated ratios in combination with molecular weight complete the description of the silicone material under the MDTQ system. Higher relative molar amounts of T, Q, T' and/or Q' to D, D', M and/or or M' in a silicone resin is indicative of higher levels of crosslinking. As discussed before, however, the overall level of crosslinking can also be indicated by the oxygen to silicon ratio.

The silicone resins for use herein which are preferred are MQ, MT, MTQ, MQ and MDTQ resins. Thus, the preferred silicone substituent is methyl. Especially preferred are MQ resins wherein the M:Q ratio is from about 0.5 : 1.0 to about 1.5 : 1.0 and the average molecular weight of the resin is from about 1,000 to about 10,000.

Further silicones for use herein which are preferred are amino functional siloxanes which conform to the general formula (III) 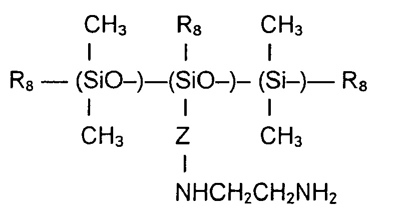 wherein R₈ = OH or CH₃ and Z represents the propyl, isopropyl or isobutyl group. These silicones, e. g., copolymer of aminoethyl aminopropyl siloxane and dimethyl siloxane are available from Dow Corning and sold under the trade name Dow Corning 2-8566 Amino Fluid^{®} or as a mixture with polyethylenglycol ether of tridecyl alcohol and cetyl trimethyl ammoniumchloride, sold as Dow Corning 929 Cationic Emulsion^{®}.

Silicone polymers that are specially preferred are CTFA: QUATERNIUM-80 and AMODIMETHICONE. Also preferred are volatile silicones such as e. g. CTFA: Dl-METHICONE and CYCLOMETHICONE.

The silicone hair conditioning agent can be used in the compositions hereof at levels of from about 0.1 % to about 40% by weight of the composition, preferably from about 0.1 % to about 20%, more preferably from about 0.5% to about 10%, more preferably from about 0.5% to about 5% and most preferably from about 0.5% to about 3.0% by weight.

The high molecular weight silicone according to the invention prevents and repairs damage such as split hair and torn hair by coating the damaged portion of the hair surface with a thin film of a high molecular weight silicone.

### Additional Conditioning Agents

The compositions of the present invention can also comprise one or more additional conditioning agents, such as those selected from the group consisting of liquid oils and fats such as avocado oil, tsubaki oil, turtle oil, Macademia nuts oil, corn oil, mink oil, olive oil, rape seed oil, yolk oil, sesame oil, parsic oil, wheat germ oil, sasanqua oil, castor oil, linseed oil, safflower oil, cotton seed oil, perilla oil, soybean oil, peanut oil, tea seed oil, kaya oil, rice bran oil, Chinese tung oil, Japanese tung oil, hohoba oil, germ oil, triglycerine, trioctanoic acid glycerine, triisopalmitic acid glycerine; solid fats such as cacao fat, coconut oil, horse fat, hardened coconut fat, palm oil, tallow, sheep fat, hardened tallow, palm kernel oil, jojoba oil, lard, ox bone fat, wood wax kernel oil, hardened castor oil; waxes such as beeswax, apple wax, canderilla wax, cotton wax, carunauba wax, bayberry wax, insect wax, whale wax, montan wax, rice bran wax, lanolin, kapok wax, lanolin acetate, liquid lanolin, cane wax, isopropyl lanolin fatty acid, hexyllaurate, reduced lanolin, jojoba wax, hard lanolin, shellac wax, POE lanolin alcohol ether, POE lanolin alcohol acetate, POE cholesterol ether, lanolin fatty acid polyethylene glycol, POE hydrogenated lanolin alcohol ether; hydrocarbons , nonvolatile hydrocarbons and hydrocarbon esters such as fluid paraffin, solid paraffin, vaseline, ozocerite, squalene, pristan, ceresin, squalane, petrolatum, microcrystalline wax; fatty acid oils, alcohols, ester oils such as cetyl octanoate, isopropyl myristate; betaine, carnitin, carnitin esters, creatine, amino acids, peptides, proteines, vitamines, phospholipides, e. g. lecithines or ceramides. Useful are also imidazolidinyl derivatives as for example (CTFA) QUATERNIUM-87 (Rewoquat^{®} W 575 of Witco, Germany).

### Fatty Alcohols

The compositions of the present invention preferably contains no fatty alcohol but may in certain cases also comprise a nonvolatile low melting point fatty alcohol. The fatty alcohols hereof have a melting point of 30°C or less, preferably about 25°C or less, more preferably about 22°C or less. The unsaturated fatty alcohols hereof are also nonvolatile. By nonvolatile what is meant is they have a boiling point at 1.0 atmospheres of at least about 260°C, preferably at least about 275°C, more preferably at least about 300°C. Suitable fatty alcohols include unsaturated monohydric straight chain fatty alcohols, saturated branched chain fatty alcohols, saturated C8-C12 straight chain fatty alcohols, and mixtures thereof. The unsaturated straight chain fatty alcohols will typically have one degree of unsaturation. Di- and tri- unsaturated alkenyl chains may be present at low levels, preferably less than about 5% by total weight of the unsaturated straight chain fatty alcohol more preferably less than about 2%, most preferably less than about 1%. Preferably, the unsaturated straight chain fatty alcohols will have an aliphatic chain size of from C12-C22, more preferably from C12-C18, most preferably from C16-C18. Exemplary alcohols of this type include oleyl alcohol, and palmitoleic alcohol. The branched chain alcohols will typically have aliphatic chain sizes of from C12-C22, preferably C14-C20, more preferably C16-C18.

Exemplary branched chain alcohols for use herein include isostearyl alcohol, octyl dodecanol, and octyl decanol.

Examples of saturated C8-C12 straight chain alcohols include octyl alcohol, caprylic alcohol, decyl alcohol, and lauryl alcohol. The low melting point fatty alcohols hereof are used at a level of from about 0.1 % to about 10%, by weight of the composition, more preferably from about 0.2% to about 5%, most preferably from about 0.5% to about 3%.

The present compositions are preferably limited to levels of monohydric saturated straight chain fatty alcohols, such as cetyl alcohol and stearyl alcohol, and other waxy fatty alcohols having melting points above 45°C, of no more than about 5%, by weight of the composition, preferably no more than about 4% since the presence of such waxy fatty alcohols can adversely affect the shine benefits of the present invention.

However, it may be desirable to use waxy fatty alcohols for their conditioning benefits. In the event that such saturated fatty alcohols are present, the weight ratio of the liquid to waxy fatty alcohols is preferably no greater than about 0.25, more preferably no greater than about 0.15, more preferably than about 0.10.

The total amount of fatty alcohols in the composition is preferably about 0.5 to about 5.0 % by weight, more preferably from about 1.0 to about 4.0% by weight, and most preferably from about 1.5 to about 3.0% by weight.

### Other Ingredients

The compositions herein can contain a variety of other optional components suitable for rendering such compositions more cosmetically or aesthetically acceptable or to provide them with additional usage benefits. Such conventional optional ingredients are well-known to those skilled in the art.

A wide variety of additonal ingredients can be formulated into the present composition. These include: hair-hold polymers, detersive surfactants such as anionic, nonionic, amphoteric, and zwitterionic surfactants; additional thickening agents and suspending agents, such as xanthan gum, guar gum, hydroxyethyl cellulose, methyl cellulose, hydroxymethylcellulose, starch and starch derivatives, viscosity modifiers such as methanolamides of long chain fatty acids, cocomonoethanol amide, salts such as sodium potassium chloride and sulfate and crystalline suspending agents, and pearlescent aids such as ethylene glycol distearate; UV-filters and sunscreens, e. g. such as p-methoxy cinnamic acid isoamylester, lipophilc cinnamic acid esters, salicylic acid esters, 4-amino benzoic acid derivatives or hydrophilic sulfonic acid derivatives of benzophenones or 3-benzyliden campher; antioxidants such as tocopheroles; agents for combating free radicals; preservatives such as benzyl alcohol, methyl paraben, propyl paraben and imidazolidinyl urea; polyvinyl alcohol; ethyl alcohol; pH adjusting agents, such as citric acid, formic acid, glyoxylic acid, acetic acid, lactic acid, pyruvic acid, sodium citrate, succinic acid, phosphoric acid, sodium hydroxide, sodium carbonate; salts, in general, such as potassium acetate and sodium chloride; coloring agents, such as any of the FD&C or D&C dyes; hair oxidizing (bleaching) agents, such as hydrogen peroxide, perborate and persulfate salts; hair reducing agents, such as the thioglycolates; perfumes, sequestering agents, such as disodium ethylenediamine tetra-acetate, and polymer plasticizing agents, such as glycerin, disobutyl adipate, butyl stearate, and propylene glycol.

Such optional ingredients generally are used individually at levels from about 0.01 % to about 10.0%, preferably from about 0.05% to about 5.0% of the composition.

### Viscosity

The hair treating composition of the present invention preferably has a viscosity at 25°C of at least about 500 mPa · s, preferably from about 500 mPas to about 10,000 mPas, more preferably from about 2,000 mPa · s to about 10,000 mPa · s. Viscosity is determined ― if not otherwise defined - by HAAKE Rotation Viscometer VT 550 with cooling/heating vessel and sensor systems according to DIN 53019 (MV-DIN), shear rate is 12.9 s⁻¹.

### METHOD OF USE

The hair care compositions of the present invention are used in conventional ways to provide the conditioning and shine benefits of the present invention. Such method of use depends upon the type of composition employed but generally involves application of an effective amount of the product to the hair, which may then be rinsed from the hair (as in the case of hair rinses) or allowed to remain on the hair (as in the case of gels, lotions, and creams). By "effective amount" is meant an amount sufficient enough to provide a hair shine benefit. In general, from about 1 g to about 50g is applied to the hair on the scalp. The composition is distributed throughout the hair typically by rubbing or massaging the hair and scalp with ones' hands or by another's hands. Preferably, the composition is applied to wet or damp hair prior to drying of the hair. After such compositions are applied to the hair, the hair is dried and styled in accordance with the desires of the user and in the usual ways of the user. Alternately, the composition is applied to dry hair, and the hair is then combed or styled in accordance with the desires of the user.

### EXAMPLES

The following examples illustrate the present invention. It will be appreciated that other modifications of the present invention within the skill of those in the hair care formulation art can be undertaken without departing from the spirit and scope of this invention.

All parts, percentages, and ratios herein are by weight unless otherwise specified. Some components may come from suppliers as dilute solutions. The levels given reflect the weight percent of the active material, unless otherwise specified.

### Example 1 Rinse out Conditioner

| | |
|---|---|
| 2.00 g | thickening system of invention |
| 1.00 g | cetyltrimethyl ammonium chloride |
| 1.00 g | polymethylphenyl siloxane (CTFA: OUATERNIUM-80; Abil Quat^{®} 3272) |
| 0.40 g | phenoxy ethanol |
| 0.20 g | PHB-methylester |
| 1.00 g | copolymer of aminoethyl aminopropyl siloxane and dimethyl siloxane emulsion as a mixture with polyethylenglycol ether of tridecyl alcohol and cetyl trimethyl ammoniumchloride (CTFA: AMODIMETHICONE & TRIDECETH-12 & CETRIMONIUM CHLORIDE; Dow Corning 949 Cationic Emulsion ^{®}) |
| 5.00 g | isododecane |
| 0.40 g | perfume oil |
| ad 100.00 g | water |
| pH = 5.85 | viscosity = 7,390 mPa · s at 25°C |

### Example 2 Leave in Conditioner

| | |
|---|---|
| 2.00 g | thickening system of invention |
| 0.50 g | 2-hydroxy-3-(trimethylamonio)propylether chloride guar gum |
| 0.50 g | sodium benzoate |
| 0.10 g | glyoxylic acid |
| 0.10 g | creatine |
| 0.80 g | behenyl trimethylammonium chloride |
| 0.60 g | cetylstearyl alcohol |
| 0.10 g | stearic acid polyethylenglycol (20 EO) |
| 0.10 g | hydrolyzed silk |
| 0.20 g | perfume oil |
| ad 100.00 g | water |
| pH = 5.2 | viscosity = 930 mPa · s at 25°C |

### Example 3 Leave in Conditioner

| | |
|---|---|
| 2.00 g | thickening system of invention |
| 0.50 g | behenyl trimethylammonium chloride |
| 0.30 g | d-panthenol |
| 0.05 g | sodium chloride |
| 10.00 g | propylene glycol |
| 0.50 g | polymethylphenyl siloxane (CTFA: OUATERNIUM-80; Abil Quat^{®} 3272) |
| 0.20 g | PHB-methylester |
| 0.10 g | PHB-propylester |
| 0.10 g | vitamine E-acetate |
| 2.00 g | isododecane |
| 0.20 g | perfume oil |
| ad 100.00 g | water |
| pH = 5.8 | viscosity = 5,556 mPa · s at 25°C |

### Example 4 Split Ends Fluid

| | |
|---|---|
| 1.00 g | thickening system of invention |
| 21.00 g | cyclo penta siloxane (CTFA: CYCLOMETHICONE) |
| 2.50 g | dihydroxy polydimethyl siloxane (CTFA: DIMETHICONOL) |
| 0.50 g | polymethylphenyl siloxane (CTFA: OUATERNIUM-80; Abil Quat^{®} 3272) |
| 0.10 g | vitamine E-acetate |
| 1.50 g | ethanol |
| 0.60 g | perfume oil |
| ad 100.00 g | water |
| pH = 6.35 | viscosity = 4,700 mPa · s at 25°C |

### Example 5 Styling Gel

| | |
|---|---|
| 0.85 g | thickening system of invention |
| 0.80 g | mixture of palmitate esters of sorbitol and sorbitol anhydrides condensed with 20 moles of ethyleneoxide (CTFA: POLYSORBATE 40) |
| 0.20 g | betaine |
| 1.50 g | polyvinyl pyrrolidone |
| 0.10 g | vitamine E-acetate |
| 1.50 g | glycerine |
| 15.00 g | ethanol |
| 0.20 g | perfume oil |
| ad 100.00 g | water |
| pH = 6.45 | viscosity = 5,240 mPa · s at 25°C |

### Example 6 Curl Refresher

| | |
|---|---|
| 3.00 g | thickening system of invention |
| 0.60 g | ethoxylated hydrogenated castor oil (PEG-40) |
| 0.25 g | ethoxylated hydrogenated castor oil (PEG-25) |
| 2.50 g | polyvinylpyrrolidone |
| 1.20 g | copolymer of metylvinylimidazolium chloride and vinylpyrrolidone (CTFA: POLYQUATERNIUM-16) |
| 0.20 g | vitamine E-acetate |
| 1.50 g | 1,2-propylenglycol |
| 0.20 g | perfume oil |
| 0.01 g | colorant |
| 1.00 g | phenoxyethanol |
| 9.00 g | ethanol |
| ad 100.00 g | water |
| pH = 5.56 | viscosity = 3,985 mPa · s at 25°C |

## Claims

1. Use of a thickening system to thicken hair treating compositions in an amount of from about 0.1 % to about 30% by weight of the composition **characterized in that** the thickening system comprises a mixture of
(i) from about 50 to about 70% of a copolymer of the ammonium salt of 2-propenoic acid with 2-propenamid;
(ii) from about 25 to about 35% of the homopolymer of 2-methyl-1-propene;
(iii) from about 3 to about 8% of poly(oxy-1,2-ethanediyl)-sorbitan-monododecanoate and
(iv) from about 0 to about 22% by weight of water.

2. Use according to claim 1, **characterized in that** said thickening system is used in an amount of from about 0.5 % to about 10% by weight of the hair treating composition.

3. Use as in claim 1 or 2, **characterized in that** said thickening system is used in an amount of from about 0. 5 % to about 5% by weight of the hair treating composition.

4. Use as in claim 1, 2 or 3 wherein said cationic surfactant, is selected from the group consisting of cetyl trimethyl ammonium salts, behenyl trimethyl ammonium salts, dimethyl ditallow ammonium salts and stearyl amidopropyl dimethylamine.

5. A hair conditioning composition that comprises
(A) from about 0.1 % to about 30% by weight of the total weight of the hair conditioning composition of a thickening system that comprises a mixture of
(i) from about 50 to about 70% of a copolymer of 2-propenoic acid with 2-propenamide;
(ii) from about 25 to about 35% of the homopolymer of 2-methyl-1-propene;
(iii) from about 3 to about 8% of poly(oxy-1,2-ethanediyl)-sorbitan-monododecanoate and
(iv) from 0 to about 22% by weight of water
and
(B) from about 0.1 % to about 10% by weight of the total weight of the hair conditioning composition of a hair conditioning agent.

6. Composition as in claim 5, **characterized in that** the said hair conditioning agent is selected from the group consisting of cationic surfactants, cationic polymers, silicones and mixtures thereof.

7. Composition as in claim 6, wherein said cationic surfactant, is selected from the group consisting of cetyl trimethyl ammonium salts, behenyl trimethyl ammonium salts, dimethyl ditallow ammonium salts and stearyl amidopropyl dimethylamine.

8. Composition as in claim 6 or 7, wherein the said silicone is selected from the group consisting of polymethylphenylsiloxane; copolymer of aminoethyl aminopropyl siloxane and dimethylsiloxane emulsion as a mixture with polyethylenglycol ether of tridecyl alcohol and cetyl trimethyl ammoniumchloride; cyclo penta siloxane; dihydroxy polydimethyl siloxane .

9. Composition as in claim 6, 7 or 8, wherein the said cationic polymer is selected from the group consisting of copolymer of metylvinylimidazolium chloride and vinylpyrrolidone.

10. Composition as in claim 1, 2, 3, 4, 5, 6, 7, 8 or 9, wherein the amount of water is from about 50 to about 98% by weight of the total weight of the hair conditioning composition.

11. A method of conditioning hair, said method comprising applying an effective amount for providing a hair care effect on the hair of the composition of claim 5, 6, 8 , 9 or 10 to the hair.
